# EUROPEAN PATENT SPECIFICATION

(11) **EP 2 552 402 B1**
(45) Date of publication and mention of the grant of the patent: **23.11.2016**
(21) Application number: 11707305.6
(22) Date of filing: 25.02.2011
(51) Int. Cl.: A61K 9/00, A61K 9/70, A61K 47/34, A61K 45/06, A61K 31/167, A61K 31/522, A61K 47/10, A61K 47/26

(54) **MULTILAYER MELT-EXTRUDED FILM**
MEHRSCHICHTIGE SCHMELZEXTRUDIERTE FOLIE
FILM MULTICOUCHE EXTRUDÉ À L'ÉTAT FONDU

(30) Priority: 26.03.2010 US 317893 P
(43) Date of publication of application: 06.02.2013
(73) Proprietor: Dow Global Technologies LLC, Midland, MI 48674 (US)
(72) Inventor: HALL, Mark, Midland, MI 48642-3150 (US); COPPENS, Karen, Midland, MI 48640 (US); READ, Michael, Midland, MI 48642 (US); SHRESTHA, Uma, Bay City, MI 48706 (US)
(74) Representative: f & e patent
(86) International application number: PCT/US2011/026230
(87) International publication number: WO 2011/119288

(56) References cited:
- WO-A2-2007/112285
- DE-A1- 19 852 826
- US-B1- 6 375 963
- ANONYMOUS: '3M(TM) CoTran(TM) 9720 Backing - Polyethylene Monolayer Film', [Online] October 2004, pages 1 - 2, XP055103263 Retrieved from the Internet: <URL:http://solutions.3m.com/3MContentRetri evalAPI/BlobServlet?lmd=1219086559000&local e=en_WW&assetType= MMM_Image&assetId=1114279699360&blobAttribu te=ImageFile> [retrieved on 2014-02-19]

## Description

### Field of the Invention

This invention relates to a multilayer melt-extruded film, a process for producing it and its use.

### Background of the Invention

Film compositions that exhibit instant wettability followed by dissolution/disintegration have been used to deliver or administer therapeutic or cosmetic substances, food flavor-imparting agents, or other ingredients within the film.

However, historically films and the process of making drug delivery systems there from have suffered from a number of unfavorable characteristics that have not allowed them to be used in practice. U.S. Patent Application Publication No. 2005/037055 discusses in detail in paragraphs [0005] - [0012] disadvantages of known films, such as agglomeration of film components which leads to an inhomogeneous distribution of the active ingredient or non-uniform films, particularly if the films are relatively thick. Non-uniform films are caused by conventional techniques for drying aqueous polymer solutions to produce a film, wherein the surface water is immediately evaporated forming a polymer film or skin. Evaporation of remaining water vapor under the surface of the film results in repeated destruction and reformation of the film surface, which is observed as a "ripple effect" which produces an uneven film. To solve these problems US 2005/037055 suggests the production of rapid-dissolving film products comprising a water-soluble polyethylene oxide alone or in combination with a hydrophilic cellulosic polymer which is free of added plasticizer. Polymer, water, and an active or other component is formed into a sheet or film by coating, spreading, casting or drawing the multi-component matrix and drying the film from the bottom of the film to the top of the film. Alternatively the film is formed by extrusion. According to the examples of US 2005/037055 rapid dissolving thin films having a content of an active ingredient of less than 5 % by weight were produced by roll coating. While the taught drying method may be useful to obtain a uniform film, US 2005/037055 does not address the problem of how to produce a film comprising a high amount of active ingredient.

WO 2005/082048 addresses the need of preparing films comprising a high amount of active ingredient, specifically caffeine at an amount of at least 18% by dry weight of the film. US 2004/0180077 discloses edible strips comprising a high amount of active ingredient. These films are prepared by making a solution of the active ingredient, a film-forming ingredient, such as a water-soluble polymer, coating the mixture on a substrate, and drying the film. However, this method of producing the films comprising a high amount of active ingredient does not solve the problem of non-uniform films that has been addressed in U.S. Patent Application Publication No. 2005/037055.

WO 2007/112285 discloses a melt-extruded film comprising: i) one or more active ingredient layers produced from a melt-extruded polymer composition comprising a polymer and an active ingredient; and ii) a removable protective layer, adjacent to the active ingredient layer. The protective layer may or may not be coextruded with the other layers.

There is still a need to provide new films. There is a particular need to provide films which can comprise a high amount of an active ingredient. There is also a particular need to produce films wherein conventional techniques for drying aqueous polymer solutions which cause the production of non-uniform films can be avoided.

### Summary of the Invention

One aspect of the present invention is a multilayer melt-extruded film comprising i) one or more active ingredient layers produced from a melt-extruded polymer composition comprising a) a water-soluble polymer which is a polyethylene oxide and b) an active ingredient, wherein the amount of the active ingredient is 5 to 60 percent, based on the total weight of the active ingredient layer i), and the active ingredient layer has a thickness of 0.15 mm or less; and ii) one or more removable protective layers to leave the active ingredient layer(s) substantially intact wherein said one or more protective layers comprises a polyolefin as film-forming polymer.

Another aspect of the present invention is a process for producing a melt-extruded film comprising the step of coextruding i) one or more active ingredient layers produced from a melt-extruded polymer composition comprising a) a water-soluble polymer which is a polyethylene oxide and b) an active ingredient, wherein the amount of the active ingredient is 5 to 60 percent, based on the total weight of the active ingredient layer i), and the active ingredient layer has a thickness of 0.15 mm or less; ii) one or more removable protective layers to leave the active ingredient layer(s) substantially intact after removal of the protective layer(s) wherein said one or more protective layers comprises a polyolefin as film-forming polymer; and iii) optionally an excipient layer between the active ingredient layer(s) and the protective layer(s).

Another aspect of the present invention is a non-therapeutic method of administering an active ingredient to an individual needing or desiring said active ingredient, said method comprising providing the multilayer melt-extruded film according to the present invention, removing one or more protective layers, optionally cutting the film into single dosage forms before or after removing one or more protective layers from the active ingredient layer and applying the active ingredient layer to a moist area of an individual, upon which application the active ingredient is released.

Another aspect of the present invention is the melt-extruded film according to the present invention for use in a method of treating an indication by administering an active ingredient to an individual needing or desiring said active ingredient, wherein the active ingredient is a drug and said method comprising providing the multilayer melt-extruded film, removing one or more protective layers, optionally cutting the film into single dosage forms before or after removing one or more protective layers from the active ingredient layer and applying the active ingredient layer to a moist area of an individual, upon which application the active ingredient is released.

Disclosed is a multilayer melt-extruded film which comprises i) one or more active ingredient layers produced from a melt-extruded polymer composition comprising a) a water-soluble polymer and b) an active ingredient; and ii) one or more removable protective layers to leave the active ingredient layer(s) substantially intact.

Also disclosed is a multilayer melt-extruded film which comprises i) one or more active ingredient layers produced from a melt-extruded polymer composition comprising a) a water-soluble polymer and b) an active ingredient; and ii) one or more protective layers being adjacent to the active ingredient layer(s) and having larger cohesive forces than adhesive forces to the active ingredient layer(s).

Also disclosed is a process for producing a melt-extruded film which comprises the step of coextruding i) one or more active ingredient layers produced from a melt-extruded polymer composition comprising a) a water-soluble polymer and b) an active ingredient; ii) one or more removable protective layers to leave the active ingredient layer(s) substantially intact after removal of the protective layer(s); and iii) optionally an excipient layer between the active ingredient layer(s) and the protective layer(s).

Also disclosed is a process for producing a melt-extruded film which comprises the step of coextruding i) one or more active ingredient layers produced from a melt-extruded polymer composition comprising a) a water-soluble polymer and b) an active ingredient; and ii) one or more protective layers having larger cohesive forces than adhesive forces to the active ingredient layer(s).

Also disclosed is a method of administering an active ingredient to an individual needing or desiring the active ingredient, which method comprises providing the above-mentioned multilayer melt-extruded film, removing one or more protective layers, optionally cutting the film into single dosage forms before or after removing one or more protective layers from the active ingredient layer and applying the active ingredient layer to a moist area of an individual, upon which application the active ingredient is released.

### Detailed Description of the Invention

The multilayer melt-extruded film comprises i) one or more active ingredient layers described further below and one or more removable protective layers which leave the active ingredient layer(s) substantially intact upon removal of the protective layer(s).

The active ingredient layer i) is produced from a melt-extruded polymer composition which generally comprises from 20 to 95 percent, preferably from 35 to 90 percent, more preferably from 40 to 85 percent and most preferably from 45 to 70 percent of a water-soluble polymer a) which is a polyethylene oxide, from 5 to 60 percent, more preferably from 10 to 45 percent, and most preferably from 20 to 40 percent of an active ingredient b) and preferably from 0 to 50 percent, more preferably from 5 to 40 percent, and most preferably from 10 to 30 percent of an optional additive c), based on the total weight of the polymer composition.

The combined amount of the water-soluble polymer a) and the active ingredient b) is preferably at least 50 percent, more preferably at least 70 percent, and most preferably at least 80 percent, based on the total weight of the polymer composition. The polymer composition can comprise one or more of the water-soluble polymers a), one or more of the active ingredients b), and one or more of the optional additives c), however their total amount is generally within the above-mentioned ranges.

The water soluble polymer a) preferably has a solubility in water of at least 1 grams, more preferably at least 3 grams, most preferably at least 5 grams in 100 grams of distilled water at 25 °C and 1 atmosphere. The water-soluble polymer a) is preferably selected from the group consisting of ethylene oxide homo- and copolymers having a weight average molecular weight of at least 10,000 or a combination of one or more of these polymers.

The water-soluble polymer generally has a weight average molecular weight of at least 15,000 g/mol, preferably at least 20,000 g/mol, more preferably at least 25,000 g/mol, most preferably at least 30,000 g/mol. The preferred upper limit for the weight average molecular weight largely depends on the type of polymer. Generally the weight average molecular weight of the water-soluble polymer is up to 10,000,000 g/mol, preferably up to 8,000,000 g/mol, more preferably up to 5,000,000 g/mol. The weight average molecular weight can be determined by light scattering according to the Standard Test Method ASTM D-4001-93 (2006).

The water-soluble polymer is a polyethylene oxide. The term "polyethylene oxide" as used herein includes homo- and copolymers of ethylene oxide. The ethylene copolymer may be a random copolymer produced by the polymerization of ethylene oxide mixed with at least one other oxide, such as 1,2-cyclohexene epoxide, 1,2-butene epoxide, allyl glycidyl ether, glycidyl methacrylate, epichlorohydrin, 1,3-butadiene diepoxide, styrene oxide, 4-vinyl-1-cyclohexene 1,2-epoxide, 4-(2-trimethoxysilylethyl)-1,2-epoxycyclohexene and 4-vinyl-1-cyclohexene diepoxide, preferably an alkylene oxide, such as propylene oxide, 1,2-butene epoxide, or isobutylene oxide. Other useful ethylene oxide copolymers are block copolymers produced by the sequential addition of ethylene oxide and at least one other alkylene oxide, in which nearly total consumption of the first monomer takes place prior to the addition of subsequent monomer(s). Alternatively, the ethylene oxide copolymer may comprise in copolymerized form ethylene oxide and another copolymerizable monomer, such as methyl acrylate, ethyl acrylate, a caprolactone, ethylene carbonate, trimethylene carbonate, 1,3-dioxolane, carbon dioxide, carbonyl sulfide, tetrahydrofuran, methyl isocyanate, or methyl isocyanide. Preferred ethylene oxide copolymers are copolymers of ethylene oxide with epichlorohydrin or copolymers of ethylene oxide with cyclohexene oxide. Ethylene oxide copolymers generally comprise at least 50 mole percent, preferably at least 70 mole percent, more preferably at least 85 mole percent ethylene oxide units. The most preferred ethylene oxide polymers are ethylene oxide homopolymers. The polyethylene oxide preferably has a weight average molecular weight of from 50,000 g/mol to 10,000,000 g/mol, more preferably from 70,000 g/mol to 8,000,000 g/mol, most preferably from 90,000 g/mol to 5,000,000 g/mol. Polyethylene oxides useful in the present composition are commercially available from The Dow Chemical Company. The average molecular weight of the polyethylene oxide employed will generally affect the processing conditions selected. A very high average molecular weight polyethylene oxide, such as greater than about 5,000,000 g/mol, will generally require higher processing temperature, torque and/or pressure in the extrusion process than a polyethylene oxide having an average molecular weight less than or equal to about 5,000,000 g/mol.

A large variety of active ingredients can be included in the composition for producing the active ingredient layer i), preferably biologically active ingredients, particularly health-related biologically active ingredients, such as vitamins, herbals and mineral supplements, oral care ingredients and drugs, but also active ingredients not directly related to health, such as flavors, colors, taste masking compounds, cosmetically active ingredients, or ingredients active in agriculture. The active ingredient includes hydrophobic, hydrophilic and amphiphilic compounds. It is not necessary for the active ingredient to be soluble in any given component of the composition. The active ingredient may be dissolved, partially dissolved or suspended in the polymer matrix of the composition. The active ingredient should generally be stable during the melt extrusion process conditions used. By stable, it is meant that a significant portion of the active ingredient will not be significantly degraded or decomposed throughout the melt extrusion process.

The active ingredients which may be incorporated in the composition for producing the active ingredient layer i) may be used for treating indications such as, by way of example and without limitation, inflammation, gout, hypercholesterolemia, microbial infection, AIDS, tuberculosis, fungal infection, amoebic infection, parasitic infection, cancer, tumor, organ rejection, diabetes, heart failure, arthritis, asthma, pain, congestion, urinary tract infections, vaginal infection, seizure related disorder, depression, psychosis, convulsion, blood coagulation, hypertension and birth control.

Examples of active ingredients that can be administered by the active ingredient layer i) are, for example, (1) analgesics such as aspirin and ketoprofen; (2) anesthetics such as lidocaine and benzocaine; (3) antiarthritics and anti-inflammatory agents such as indomethacin, dexamethasone, ibuprofen, allopurinol, hydrocortisone, betamethasone, dexamethasone, prednisolone and indomethacin; (4) antiasthma drugs such as theophylline, ephedrine, beclomethasone and epinephrine; (5) urinary tract disinfectives such as sulfarmethoxazole and trimethoprim; (6) anticoagulants such as heparin; (7) anticonvulsants such as diazepam; (8) antidepressants such as amitriptyline and imipramine; (9) agents useful in the treatment of diabetics and regulation of blood sugar; (10) antineoplastics such as adriamycin, fluorouracil and methotrexate; (11) antipsychotics; (12) antihypertensives such as methyldopa, clonidine, timolol, propranolol, prazosin hydrochloride and reserpine; (13) muscle relaxants such as mephalan and diazepam; (14) antiprotozoals such as chloramphenicol and trimethoprim; (15) spermicidals; (16) antibacterial substances, tetracyclines, chloramphenicol and neomycin; (17) antihistamines and decongestants such as chlorpheniramine, pseudophedrine and phenylephrine; (18) antiparasitic compounds; and (19) antiviral compounds such as acyclovir.

Other preferred drugs for other preferred active ingredients for use in the present invention include anti-diarrheals such as immodium AD, anti-histamines, anti-tussives, decongestants, vitamins, and breath fresheners. Common drugs used alone or in combination for colds, pain, fever, cough, congestion, runny nose and allergies, such as acetaminophen, chlorpheniramine maleate, dextromethorphan, pseudoephedrine HCl and diphenhydramine may be included in the film compositions of the present invention.

Cosmetic active agents may include breath freshening compounds like menthol, other flavors or fragrances, especially those used for oral hygiene, as well as actives used in dental and oral cleansing such as quaternary ammonium bases. The effect of flavors may be enhanced using flavor enhancers like tartaric acid, citric acid, vanillin, or the like. Examples of the range of such nutritional supplements usable in the invention include, but are not limited to, Cherry extract, Ginkgo biloba extract, Kava Kava extract, Ginseng extract, Saw Palmetto extract, cranberry or blueberry extract, tomato extract, cordyceps sinensis extract, pomegranates, elderberries, as well as the entire berry family, strawberry, raspberry, cherry, black raspberry, boysenberry, etc., glucosamine sulfate, chromium picolinate, Milk thistle extract, Grape seed extract, Ma Huang extract, Co-enzyme Q10, water soluble vitamins such as vitamin C niacin, vitamin B1 and vitamin B12, and fat soluble vitamins such as vitamins A, D, E, and K, minerals such as calcium, magnesium and zinc, among others.

Examples of active ingredients which are particularly suitable for including in the polymer composition to be melt extruded are ibuprofen (as racemate, enantiomer or enriched enantiomer), ketoprofen, flurbiprofen, acetylsalicylic acid, verapamil, paracetamol, nifedipine, captopril, omeprazole, ranitidine, tramadol, cyclosporin, trandolapril and therapeutic peptides.

The composition to be melt-extruded may comprise one or more optional additives c), such as one or more disintegrants, fillers, pigments, colorants, lubricants, plasticizers, stabilizers such as antioxidants, slip agents and anti-block agents. However, one advantage of the present invention is that it is not necessary to incorporate one or more lubricants or plasticizers or stabilizers or slip agents or anti-block agents in the polymer composition to be melt-extruded for preparing the active ingredient layer i).

A disintegrant can be incorporated as optional additive c) in the composition to be melt-extruded to reduce the disintegration or dissolution time of the produced melt-extruded film. Useful disintegrants are, as examples but not limited to, mono- and disaccharides, sugar alcohols, salts of cross-linked carboxymethylcellulose and water soluble polymers with a lower molecular weight than the water-soluble polymer a).

The compositions of a), b) and optionally c) described herein are generally melt-extrudable. As used herein, the term "melt-extrudable" refers to a compound or composition that may be melt-extruded, particularly hot-melt extruded. A hot-melt extrudable polymer composition is one that is sufficiently rigid at 25°C and atmospheric pressure, when it is not in particulate form such as a powder or granules, but is capable of deformation or forming a semi-liquid state under elevated heat or pressure, that means at a temperature above 25 °C or a pressure above atmospheric pressure. Although the polymer composition utilized for producing the active ingredient layer i) need not contain a plasticizer to render it hot-melt extrudable, a plasticizer may be included as an additional component. The plasticizer should be able to lower the glass transition temperature or softening point of the active composition in order to allow for lower processing temperature, extruder torque and pressure during the hot-melt extrusion process. Plasticizers also generally reduce the viscosity of a polymer melt thereby allowing for lower processing temperature and extruder torque during hot-melt extrusion. Useful plasticizers are, for example, cetanol, triglycerides, polyoxyethylene-polyoxypropylene glycol (Pluronic), triacetin or triethyl citrate. Plasticizers are advantageously included when a water-soluble polymer of very high molecular weight such as greater than about 5,000,000 g/mol is employed.

The melt-extruded film of the present invention comprises one or more active ingredient layers. Each of the active ingredient layers has a composition as described above, but the active ingredient layers may be different from each other. For example if the melt-extruded film comprises more than one active ingredient layer, each active ingredient layer may comprise a different type or amount of active ingredient and/or a different type or amount of water-soluble polymer. For illustration purposes only and not limited to these embodiments, the melt-extruded film may comprise I) two active ingredient layers, each comprising a different drug or II) two active ingredient layers, one comprising a drug and the other one comprising a flavor, a color or a taste making ingredient.

It has been found that due to the co-extrusion of an active ingredient layer i) and one or more protective layers ii) the active ingredient layer i) can have a high load of the active ingredient b), typically from 10 to 45 percent, and more typically from 20 to 40 percent, based on the total weight of the active ingredient layer i), and that an even and cohererent layer i) can still be produced which results in a film of good quality with an evenly distributed content of the active ingredient after said one or more protective layers have been removed. Under optimized conditions even a higher load of the active ingredient can be achieved, such as up to 80 percent, generally up to 60 percent, based on the total weight of the active ingredient layer i). Moreover, the present invention allows minimizing the amount of the water-soluble polymer a). Surprisingly, a film of such quality can generally be produced even if the active ingredient layer has a thickness of 0.15 mm or less, under preferred conditions 0.09 mm or less, and under optimal conditions and depending on the specific composition of the active ingredient layer i), 0.05 mm or less or even 0.025 mm or less. Thus the thickness of the active ingredient layer i) generally is from 0.01 to 0.15 mm, preferably from 0.025 to 0.15 mm, most preferably from 0.08 to 0.13 mm. The resulting film is advantaged in that a given area of film can comprise a high concentration of active ingredient, thus fewer film strips are required to provide a therapeutic dose. Further, a higher active ingredient concentration in the film provides faster availability of the active ingredient as less polymer must be dissolved before the film disintegrates. Moreover, due to the co-extrusion of an active ingredient layer i) and one or more protective layers ii), a disintegrant can be incorporated in the active ingredient layer i) and an even and cohererent layer i) can still be produced which results in a film of good quality with fast disintegration and dissolution properties after said one or more protective layers have been removed.

In one aspect of the present invention the one or more protective layers are prepared from a melt-extrudable material that is useful for preparing a removable film that leaves the active ingredient layer(s) substantially intact upon removal of the protective layer(s). In general this means that no substantial damage like holes should be visible in the active ingredient layer(s) upon removal of the protective layer(s). Preferably also said one or more protective layers remain substantially intact upon removal of the protective layer(s).

Preferably said one or more protective layers are adjacent to the active ingredient layer(s).

Alternatively, an excipient layer is arranged between the active ingredient layer(s) and the protective layer(s), as described further below. In this case it is preferable that the one or more protective layers are prepared from a melt-extrudable material that is useful for preparing a removable film that also leaves the excipient layer(s) substantially intact upon removal of the protective layer(s).

In another aspect of the present invention the one or more protective layers are prepared from a melt-extrudable material that is useful for preparing a film with larger cohesive forces than its adhesive forces to an adjacent active ingredient layer i). Preferably said one or more protective layers is peelable off the active ingredient layer(s).

More preferably said one or more protective layers comprise a polyolefin, preferably a propylene-based polymer or an ethylene-based polymer as film-forming polymer.

Propylene-based polymers suitable for use in the one or more protective layers, include, but are not limited to, propylene homopolymers, propylene/ethylene copolymers, propylene/ethylene/1-butene interpolymers, propylene/ethylene/1-hexene interpolymers, and propylene/ethylene/1-octene interpolymers. Suitable propylene-based interpolymers include VERSIFY polymers (available from The Dow Chemical Company).

Ethylene-based polymers for use in the one or more protective layers include, but are not limited to, high density polyethylene (HDPE), linear low density polyethylene (LLDPE), ultra low density polyethylene (ULDPE), homogeneously branched linear ethylene polymers, and homogeneously branched substantially linear ethylene polymers (that is homogeneously branched long chain branched ethylene polymers).

High density polyethylene typically has a density of about 0.94 to about 0.97 g/cc. Typically the low density polyethylene (LDPE) is made under high-pressure, using free-radical polymerization conditions. Low density polyethylene typically has a density from 0.91 to 0 94 g/cc.

Linear low density polyethylene (LLDPE) is characterized by little, if any, long chain branching, in contrast to conventional LDPE. The processes for producing LLDPE are well known in the art and commercial grades of this polyolefin resin are available. Generally, LLDPE is produced in gas-phase fluidized bed reactors or liquid phase solution process reactors, using a Ziegler-Natta catalyst system. The linear low density polyethylene (LLDPE), ultra low density polyethylene (ULDPE). homogeneously branched linear ethylene interpolymers, or homogeneously branched substantially linear ethylene interpolymers, typically have polymerized therein at least one α-olefin. The term "interpolymer" used herein indicates the polymer can be a copolymer, a terpolymer or any polymer having more than one polymerized monomer. Monomers usefully copolymerized with ethylene to make the interpolymer include the C3-C20 α-olefins, and especially propylene, 1-butene, 1- pentene, 1-hexene, 4- methyl- 1-pentene, 1-heptene and 1-octene. Especially preferred comonomers include propylene, 1-butene, 1-hexene and 1-octene.

Commercial examples of suitable ethylene-base interpolymers include ATTANE, AFFINITY, DOWLEX, ELITE, all available from The Dow Chemical Company; and EXCEED and EXACT available from Exxon Chemical Company.

The terms "homogeneous" and "homogeneously-branched" are used in reference to an ethylene/α-olefin interpolymer, in which the α-olefin comonomer is randomly distributed within a given polymer molecule, and substantially all of the polymer molecules have the same ethylene-to-comonomer ratio. The homogeneously branched ethylene interpolymers that can be used in the practice of this invention include linear ethylene interpolymers, and substantially linear ethylene interpolymers. Included amongst the homogeneously branched linear ethylene interpolymers are ethylene polymers, which lack long chain branching (or measurable amounts of), but do have short chain branches, derived from the comonomer polymerized into the interpolymer, and which are homogeneously distributed, both within the same polymer chain, and between different polymer chains. That is, homogeneously branched linear ethylene interpolymers lack long chain branching, just as is the case for the linear low density polyethylene polymers or linear high density polyethylene polymers, made using uniform branching distribution polymerization processes, as described, for example, by Elston in U.S. Patent 3,645,992. Commercial examples of homogeneously branched linear ethylene/α-olefin interpolymers include TAFMER polymers supplied by the Mitsui Chemical Company and EXACT polymers supplied by ExxonMobil Chemical Company.

Substantially linear ethylene interpolymers are described in U.S. Patent Nos. 5,272,236; 5,278,272; 6,054,544; 6,335,410 and 6,723,810. The substantially linear ethylene interpolymers are those in which the comonomer is randomly distributed within a given interpolymer molecule, and in which substantially all of the interpolymer molecules have the same ethylene/comonomer ratio within that interpolymer. In addition, the substantially linear ethylene interpolymers are homogeneously branched ethylene interpolymers having long chain branching. The long chain branches have the same comonomer distribution as the polymer backbone, and can have about the same length as the length of the polymer backbone. "Substantially linear", typically, is in reference to a polymer that is substituted, on average, with 0.01 long chain branches per 1000 carbons to 3 long chain branches per 1000 carbons. The length of a long chain branch is longer than the carbon length of a short chain branch formed from the incorporation of one comonomer into the polymer backbone. Some polymers may be substituted with 0.01 long chain branches per 1000 carbons to 1 long chain branch per 1000 carbons, or from 0.05 long chain branches per 1000 carbons to 1 long chain branch per 1000 carbons, or from 0.3 long chain branches per 1000 carbons to 1 long chain branch per 1000 carbons. Commercial examples of substantially linear polymers include the ENGAGE polymers and AFFINITY polymers (both available from The Dow Chemical Company).

The polyolefin material that can be used for producing the protective layer(s) can comprise additional additives, such as slip, anti-block, and polymer processing aids. The thickness of each protective layer individually is preferably from 0.025 to 0.125 mm, more preferably from 0.03 to 0.10 mm, most preferably from 0.05 to 0.08 mm.

In one aspect of the present invention an excipient layer is arranged between the active ingredient layer(s) and the protective layer(s). The excipient layer is preferably produced from a melt-extruded polymer composition which generally comprises from 30 to 100 percent, preferably from 40 to 95 percent, more preferably from 60 to 90 percent of a water-soluble polymer a) as described further above and generally from 0 to 70 percent, preferably from 5 to 60 percent, and more preferably from 10 to 40 percent of an optional additive c) as described further above, based on the total weight of the polymer composition. The thickness of the excipient layer generally is from 0.01 to 0.18 mm, preferably from 0.025 to 0.15 mm, more preferably from 0.05 to 0.13 mm. The excipient layer(s) typically adheres to the active ingredient layer(s) upon removal of the protective layer(s).

The components and any additional additives of the melt-extrudable material for each layer may be pre-mixed before feeding the blend into a device utilized for melt-extrusion. Useful devices for melt-extrusion, specifically useful extruders, are known in the art. Alternatively, the components and any additional additives may be fed separately into the extruder and blended in the device before or during a heating step. Although in some embodiments of the invention the composition or the components to be fed into the extruder may contain liquid materials, dry feed is advantageously employed in the melt-extrusion process of the present invention.

The blend or the components that has or have been fed into an extruder are passed through a heated area of the extruder at a temperature which will melt or soften the mixture or at least one or more components thereof to form a blend throughout which the active ingredient is dispersed. Typical extrusion processing temperatures are from 50 to 210 °C, preferably from 70 to 200 °C, more preferably from 100 to 190°C. An operating temperature range should be selected that will minimize the degradation or decomposition of the active ingredient and other components of the blend during processing. The extruder(s) used to practice the invention preferably is a commercially available model equipped to handle dry feed and having a solid conveying zone, one or multiple heating zones, and an extrusion die. It is particularly advantageous for the extruder to possess multiple separate temperature controllable heating zones. Useful devices for conducting multilayered film coextrusion are known in the art.

For illustration purposes only and not limited to these embodiments, some of the most useful structures of the multilayer melt-extruded film of the present invention are listed herein. The multilayer melt-extruded film of the present invention can have a bi-layer structure; i.e., protective layer / active ingredient layer. More preferably, the multilayer melt-extruded film of the present invention has a three-layer structure; i.e., protective layer / active ingredient layer / protective layer, wherein the two protective layers can be the same or different. Alternatively, the multilayer melt-extruded film of the present invention can have a four-layer structure; i.e., protective layer / active ingredient layer I / active ingredient layer II / protective layer, wherein the two protective layers can be the same or different. Another useful multilayer melt-extruded film has a five- or six-layer structure; i.e., protective layer / excipient layer / active ingredient layer I / optionally active ingredient layer II / excipient layer / protective layer, wherein the two excipient layers and protective layers can be the same or different.

After the preparation of the multilayer melt-extruded film of the present invention, one or more protective layers can be removed from the active layer(s), for example by peeling the protective layer(s) off the active ingredient layer(s) or the excipient layer(s).

The mono- or multilayered film can be cut into single dosage forms according to a known manner before or after removing one or more protective layers from the active ingredient layer. The active ingredient layer is obtained in the form of a coherent film. The active ingredient layer, preferably after having it cut into single dosage forms, can be applied to a moist area of an individual who needs or desires the active ingredient in the active ingredient layer. The moist area of the individual can, for example, be the tongue or a tissue like the skin. Upon application of the active ingredient layer to the moist area of an individual the active ingredient is released.

The present invention is further illustrated by the following examples which are not to be construed to limit the scope of the invention. Unless otherwise mentioned, all parts and percentages are by weight.

### Examples 1 - 7

The protective layers of Examples 1-5 were produced of Low Density Polyethylene (LDPE) 4005, having a density of 0.918 g/cc at 23°C, measured according to ASTM D792 and a melt mass flow rate (MFR) 5.5 g/10 min. at 190°C/2.16 kg, measured according to ASTM D1238.

In Examples 6 and 7, protective layers were produced of Low Density Polyethylene (LDPE) 722, having a density of 0.918 g/cc at 23°C, measured according to ASTM D792 and a melt mass flow rate (MFR) 8.0 g/10 min. at 190°C/2.16 kg, measured according to ASTM D1238.

The active ingredient layer i) was produced from a formulation listed in Table 1 below. All components of each formulation except the flavorant spearmint were thoroughly mixed in a laboratory scale V-Blender and then in a Hobart mixer before feeding the blend into the extruder. The flavorant spearmint was only added to the Hobart mixer.

The water soluble polymers used in the formulations were POLYOX™ WSR N-10 poly(ethylene oxide) polymer which is commercially available from The Dow Chemical Company having a molecular weight of about 100,000 g/mol.

**Table 1: Composition of active ingredient layer**

| Components (weight percent) | | | | |
|---|---|---|---|---|
| | **I** | **II** | **III** | **IV** |
| Caffeine (model drug) | 35 | 35 | -- | -- |
| Acetaminophen (model drug) | -- | -- | 40 | 50 |
| Mannitol (sweetener and diluent) | 7.1 | 7.1 | -- | -- |
| POLYOX™ WSR N-10 | 49.8 | 52.5 | 60 | 50 |
| Sucralose (artificial sweetener) | 2.9 | 2.9 | -- | -- |
| Menthol (flavorant, powdered) | 2.5 | 2.5 | -- | -- |
| Spearmint | 0.2 | -- | -- | -- |
| FD&C Blue No. 1 (dye, powdered) | 0.2 | -- | -- | -- |

In Examples 1-5, multilayer coextrusion was performed using a multilayer coextrusion line. The line has three one inch diameter extruders (25.4 mm), each with a length/diameter (L/D) ratio of 24/1 and a maximum screw speed of 100 rpm. Extruder C fed the core layer while extruders A and B fed the two protective layers. A three-layer symmetrical film was produced with the active ingredient layer i) being the core layer. Extruders A and B were Killion model KTS-100 extruders, while Extruder C was a Killion model KL-100 extruder. The film was produced on a 10-inch (254 mm) wide hanger style cast film die. The film was collected on a 12-inch (305 mm) diameter casting roll assembly fabricated by Killion. The film samples were collected on cores of 1.5 inch (38 mm) diameter. The protective layers were mechanically peeled off the active ingredient layer. The thickness of the layers was measured using a micrometer.

The conditions in the extrusion trial are given in Tables 2 - 4 below:

**Table 2:**

| Example | **Example 1** | | | **Example 2** | | |
|---|---|---|---|---|---|---|
| Extruder | A | B | C | A | B | C |
| Formulation | LDPE 4005 | LDPE 4005 | Formulation No. I | LDPE 4005 | LDPE 4005 | Formulation No. I |
| Extruder (rpm) | 75 | 75 | 40 | 75 | 75 | 40 |
| Extruder Zone 1(°C) | 105 | 105 | 60 | 105 | 105 | 60 |
| Extruder Zone 2(°C) | 120 | 120 | 120 | 120 | 120 | 120 |
| Extruder Zone 3(°C) | 130 | 130 | 125 | 130 | 130 | 125 |
| Clamp Ring (°C) | 130 | 130 | 138 | 130 | 130 | 138 |
| Feedblock (°C) | -- | 140 | 138 | -- | 140 | 138 |
| Die Zone 1 (°C) | 125 | 140 | -- | 125 | 140 | -- |
| Die Zone 2 (°C) | -- | 140 | -- | -- | 140 | -- |
| Die Zone 3 (°C) | -- | 140 | -- | -- | 140 | -- |
| Cast Roll (m/min.) | -- | 1.5 | -- | -- | 6.1 | -- |
| Film thickness | 0.191 mm | 0.191mm | 0.025 mm | 0.076 mm | 0.076 mm | 0.017 mm |

**Table 3:**

| Example | **Example 3** | | | **Example 4** | | |
|---|---|---|---|---|---|---|
| Extruder | A | B | C | A | B | C |
| Formulation | LDPE 4005 | LDPE 4005 | Formulation No. I | LDPE 4005 | LDPE 4005 | Formulation No. I |
| Extruder | 75 | 75 | 40 | 75 | 75 | 40 |

| (rpm) | | | | | | |
|---|---|---|---|---|---|---|
| Extruder Zone 1 (°C) | 105 | 105 | 60 | 105 | 105 | 60 |
| Extruder Zone 2 (°C) | 120 | 120 | 120 | 120 | 120 | 120 |
| Extruder Zone 3 (°C) | 140 | 140 | 125 | 140 | 140 | 125 |
| Clamp Ring (°C) | 140 | 140 | 138 | 140 | 140 | 138 |
| Feedblock (°C) | - | 140 | 138 | - | 140 | 138 |
| Die Zone 1 (°C) | 140 | 140 | 140 | 140 | 140 | - |
| Die Zone 2 (°C) | -- | 140 | - | - | 140 | - |
| Die Zone 3 (°C) | -- | 140 | - | - | 140 | - |
| Cast Roll (m/min.) | | 6.1 | - | | 4.6 | |
| Film thickness | 0.064 mm | 0.064 mm | 0.015 mm | 0.102 mm | 0.102 mm | 0.018 mm |

**Table 4:**

| Example | **Example 5** | | |
|---|---|---|---|
| Extruder | A | B | C |
| Formulation | LDPE 4005 | LDPE 4005 | Formulation I |
| Extruder (rpm) | 75 | 75 | 40 |
| Extruder Zone 1 (°C) | 105 | 105 | 60 |
| Extruder Zone 2 (°C) | 120 | 120 | 120 |
| Extruder Zone 3 (°C) | 140 | 140 | 125 |
| Clamp Ring (°C) | 140 | 140 | 138 |
| Feedblock (°C) | -- | 140 | 138 |
| Die Zone 1 (°C) | 140 | 140 | -- |
| Die Zone 2 (°C) | -- | 140 | -- |
| Die Zone 3 (°C) | -- | 140 | -- |
| Cast Roll (m/min.) | | 7.6 | |
| Film thickness | 0.064 mm | 0.064 mm | 0.014 mm |

After removal of the protective layer, the active ingredient layer was obtained as an even and coherent film. Its thickness was 0.025 mm or less. Providing an even and coherent film of such a low thickness, although it contains a high concentration (35 weight percent) of a model drug (caffeine), solves a long-felt need in the industry.

The single screw coextrusion line used to produce Examples 6 and 7 consisted of two 31.75 mm (1.25 inch) diameter, 24:1 L/D Killion single screw extruders. The extruders each fed a Maag model 2213 gear pump to ensure uniform flow of the polymer melts to a Cloeren feedblock and a 203.2 mm (8 inch) coat-hanger style film die. The feedblock split the flow of the material from the extruder with the polyethylene layer (extruder A) to provide a coextruded structure with protective skins on the outer layer and the core layer was produced with the material from extruder B. The gear pumps were attached to a feedblock by transfer lines that contained variable depth thermocouples to ensure consistent and uniform temperatures from the extruders.

The film was extruded vertically onto a single steel casting roll with a temperature control system (maintained at 25C). The film was conveyed via a driven nip roll to a film winding station. The extrusion conditions are listed in Table 5.

**Table 5**

| Examples | **Example 6** | | **Example 7** | |
|---|---|---|---|---|
| Extruder | A | B | A | B |
| Formulation | LDPE 722 | Formulation No. III | LDPE 722 | Formulation No. IV |
| Extruder (rpm) | 100 | 30 | 100 | 30 |
| Extruder Zone 1 (°C) | 105 | 60 | 105 | 60 |
| Extruder Zone 2 (°C) | 130 | 120 | 120 | 120 |
| Extruder Zone 3 (°C) | 140 | 125 | 130 | 125 |
| Clamp Ring (°C) | 140 | 125 | 130 | 125 |
| Feedblock (°C) | -- | 140 | -- | 140 |
| Die Zone 1 (°C) | 140 | -- | 140 | -- |
| Film thickness | 0.076 mm | 0.025 mm | 0.076 mm | 0.025 mm |

After removal of the protective layer, the active ingredient layer was obtained as an even and coherent film. Its thickness was 0.025 mm or less. Providing an even and coherent film of such a low thickness, although it contains a high concentration (up to 50 weight percent) of a model drug (acetaminophen), solves a long-felt need in the industry.

Examples 1-7 illustrate that surprisingly films of good quality can be achieved even with a low molecular weight water soluble polymer, due to the good melt strength contribution of the protective layer polymer. Even more surprisingly such films based on a low molecular weight water soluble polymer can be produced of good quality, even when they have a high concentration of the active ingredient, which results in fast dissolution of the film due to low concentration of the water soluble polymer in the film.

### Comparative Example

A single screw extrusion line used to produce the comparative example consisted of a 31.75 mm (1.25 inch) diameter, 24:1 L/D Killion single screw extruder. The extruder fed a Maag model 2213 gear pump to ensure uniform flow of the polymer melt to a Cloeren feedblock and a 203.2 mm (8 inch) coat-hanger style film die. The gear pump was attached to a feedblock by a transfer line that contained variable depth thermocouples to ensure consistent and uniform temperatures from the extruder.

The film was extruded vertically onto a single steel casting roll with a temperature control system (maintained at 25C). The film was conveyed via a driven nip roll to a film winding station. The extrusion conditions are listed in Table 6.

It was not possible to produce a film of 0.127 mm thickness with formulation II. Film breakage and many holes were observed in the Comparative Example. The standard single layer film process was not capable of producing the desired thickness.

**Table 6**

| **Comparative Example** | |
|---|---|
| Formulation | II |
| Mass rate {kg/hr) | 2.5 |
| Extruder Zone 1 (°C) | 70 |
| Extruder Zone 2 (°C) | 120 |
| Extruder Zone 3 (°C) | 140 |
| Die Zone 1 (°C) | 140 |
| Die Zone 2 (°C) | 140 |
| Screw speed [rpm] | 30 |
| Cast Roll (m/min.) | 1.5 |

Examples 1-7 and the Comparative Example illustrate that multilayer coextrusion incorporating protective skin layers allows the preparation of films with active ingredient concentrations that cannot be produced via conventional monolayer extrusion.

## Claims

1. A multilayer melt-extruded film comprising
i) one or more active ingredient layers produced from a melt-extruded polymer composition comprising a) a water-soluble polymer which is a polyethylene oxide and b) an active ingredient, wherein the amount of the active ingredient is 5 to 60 percent, based on the total weight of the active ingredient layer i), and the active ingredient layer has a thickness of 0.15 mm or less; and
ii) one or more removable protective layers to leave the active ingredient layer(s) substantially intact wherein said one or more protective layers comprises a polyolefin as film-forming polymer.

2. The multilayer melt-extruded film of claim 1 wherein said one or more protective layers are adjacent to the active ingredient layer(s).

3. The multilayer melt-extruded film of claim 1 wherein an excipient layer is arranged between the active ingredient layer(s) and the protective layer(s).

4. The melt-extruded film of any one of claims 1 to 3 wherein the active ingredient layer has a thickness of 0.05 mm or less.

5. The melt-extruded film of any one of claims 1 to 4 wherein the amount of the active ingredient is 20 to 40 percent, based on the total weight of the active ingredient layer i).

6. The melt-extruded film of any one of claims 1 to 5 wherein said one or more protective layers comprises a propylene-based polymer or an ethylene-based polymer as film-forming polymer.

7. The melt-extruded film of any one of claims 1 to 6 in the form of a single dosage form.

8. A process for producing a melt-extruded film comprising the step of coextruding
i) one or more active ingredient layers produced from a melt-extruded polymer composition comprising a) a water-soluble polymer which is a polyethylene oxide and b) an active ingredient, wherein the amount of the active ingredient is 5 to 60 percent, based on the total weight of the active ingredient layer i), and the active ingredient layer has a thickness of 0.15 mm or less;
ii) one or more removable protective layers to leave the active ingredient layer(s) substantially intact after removal of the protective layer(s) wherein said one or more protective layers comprises a polyolefin as film-forming polymer; and
iii) optionally an excipient layer between the active ingredient layer(s) and the protective layer(s).

9. The process of claim 8 comprising the additional step of cutting the film into single dosage forms before or after removing one or more protective layers from the active ingredient layer(s).

10. A non-therapeutic method of administering an active ingredient to an individual needing or desiring said active ingredient, said method comprising providing the multilayer melt-extruded film of any one of claims 1 to 7, removing one or more protective layers, optionally cutting the film into single dosage forms before or after removing one or more protective layers from the active ingredient layer and applying the active ingredient layer to a moist area of an individual, upon which application the active ingredient is released.

11. The method of claim 10, wherein the active ingredient is a cosmetically active ingredient.

12. The melt-extruded film of any one of claims 1 to 7 for use in a method of treating an indication by administering an active ingredient to an individual needing or desiring said active ingredient, wherein the active ingredient is a drug and said method comprising providing the multilayer melt-extruded film, removing one or more protective layers, optionally cutting the film into single dosage forms before or after removing one or more protective layers from the active ingredient layer and applying the active ingredient layer to a moist area of an individual, upon which application the active ingredient is released.

13. The melt-extruded film for the use of claim 12, wherein the indication is selected from the group consisting of inflammation, gout, hypercholesterolemia, microbial infection, AIDS, tuberculosis, fungal infection, amoebic infection, parasitic infection, cancer, tumor, organ rejection, diabetes, heart failure, arthritis, asthma, pain, congestion, urinary tract infections, vaginal infection, seizure related disorder, depression, psychosis, convulsion, blood coagulation, hypertension and birth control.

14. The melt-extruded film for the use of claims 12 or 13, wherein the active ingredient is selected from the group consisting of 1) analgesics, preferably from the group consisting of aspirin and ketoprofen, 2) anesthetics, preferably from the group consisting of lidocaine and benzocaine, 3) antiarthritics and anti-inflammatory agents, preferably from the group consisting of indomethacin, dexamethasone, ibuprofen, allopurinol, hydrocortisone, betamethasone, dexamethasone, prednisolone and indomethacin, 4) antiasthma drugs, preferably from the group consisting of theophylline, ephedrine, beclomethasone and epinephrine, 5) urinary tract disinfectives, preferably from the group consisting of sulfarmethoxazole and trimethoprim, 6) anticoagulants, preferably heparin, 7) anticonvulsants, preferably diazepam, 8) antidepressants, preferably from the group consisting of amitriptyline and imipramine, 9) agents useful in the treatment of diabetics and regulation of blood sugar, 10) antineoplastics, preferably from the group consisting of adriamycin, fluorouracil and methotrexate, 11) antipsychotics, 12) antihypertensives, preferably from the group consisting of methyldopa, clonidine, timolol, propranolol, prazosin hydrochloride and reserpine, 13) muscle relaxants, preferably from the group consisting of mephalan and diazepam, 14) antiprotozoals, preferably from the group consisting of chloramphenicol and trimethoprim, 15) spermicidals, 16) antibacterial substances, tetracyclines, chloramphenicol and neomycin, 17) antihistamines and decongestants, preferably from the group consisting of chlorpheniramine, pseudophedrine and phenylephrine, 18) antiparasitic compounds, and 19) antiviral compounds, preferably acyclovir.

## Patentansprüche

1. Ein mehrschichtiger schmelzextrudierter Film umfassend
i) eine oder mehrere Wirkstoffstoffschichten, hergestellt aus einer schmelzextrudierten Polymerzusammensetzung umfassend a) ein wasserlösliches Polymer, welches ein Polyethylenoxid ist, und b) einen Wirkstoff, wobei die Menge des Wirkstoffs 5 bis 60 %, bezogen auf das Gesamtgewicht der Wirkstoffschicht i) ist, und die Wirkstoffschicht eine Dicke von 0,15 mm oder weniger hat; und
ii) eine oder mehrere entfernbare Schutzschicht(en), um die Wirkstoffschicht(en) im Wesentlichen unversehrt zu belassen, wobei die eine oder mehreren Schutzschicht(en) ein Polyolefin als filmbildendes Polymer umfasst/umfassen.

2. Der mehrschichtige schmelzextrudierte Film gemäß Anspruch 1, wobei die eine oder mehreren Schutzschicht(en) an die Wirkstoffschicht(en) angrenzend sind.

3. Der mehrschichtige schmelzextrudierte Film gemäß Anspruch 1, wobei eine Bindemittelschicht zwischen der (den) Wirkstoffschicht(en) und der (den) Schutzschicht(en) angeordnet ist.

4. Der schmelzextrudierte Film gemäß einem der Ansprüche 1 bis 3, wobei die Wirkstoffschicht eine Dicke von 0,05 mm oder weniger hat.

5. Der schmelzextrudierte Film gemäß einem der Ansprüche 1 bis 4, wobei die Menge an Wirkstoff 20 bis 40 %, bezogen auf das Gesamtgewicht der Wirkstoffschicht i), ist.

6. Der schmelzextrudierte Film gemäß einem der Ansprüche 1 bis 5, wobei die eine oder mehreren Schutzschicht(en) ein propylenbasiertes Polymer oder ein ethylenbasiertes Polymer als filmbildendes Polymer umfasst/ umfassen.

7. Der schmelzextrudierte Film gemäß einem der Ansprüche 1 bis 6 in der Form einer Einzeldarreichungsform.

8. Ein Verfahren zur Herstellung eines schmelzextrudierten Films umfassend den Schritt des Coextrudierens
i) einer oder mehrerer Wirkstoffschicht(en), hergestellt aus einer schmelzextrudierten Polymerzusammensetzung, umfassend a) ein wasserlösliches Polymer, welches ein Polyethylenoxid ist, und b) einen Wirkstoff, wobei die Menge an Wirkstoff 5 bis 60 %, bezogen auf das Gesamtgewicht der Wirkstoffschicht i), ist, und die Wirkstoffschicht eine Dicke von 0,15 mm oder weniger hat;
ii) einer oder mehrerer entfernbarer Schutzschicht(en), um die Wirkstoffschicht(en) nach Entfernen der Schutzschicht(en) im Wesentlichen unversehrt zu belassen, wobei die eine oder mehreren Schutzschicht(en) ein Polyolefin als filmbildendes Polymer umfasst/umfassen; und
iii) wahlweise einer Bindemittelschicht zwischen der (den) Wirkstoffschicht(en) und der (den) Schutzschicht(en).

9. Das Verfahren gemäß Anspruch 8 umfassend den zusätzlichen Schritt des Zuschneidens des Films zu Einzeldarreichungsformen vor oder nach Entfernen einer oder mehrerer Schutzschichten von der (den) Wirkstoffschicht(en).

10. Ein nicht-therapeutisches Verfahren zur Verabreichung eines Wirkstoffs an ein Individuum, das den entsprechenden Wirkstoff benötigt oder wünscht, wobei das Verfahren das Bereitstellen des mehrschichtigen schmelzextrudierten Films gemäß einem der Ansprüche 1 bis 7, das Entfernen einer oder mehrerer Schutzschicht(en), wahlweise das Zuschneiden des Films zu Einzeldarreichungsformen vor oder nach Entfernen einer oder mehrerer Schutzschicht(en) von der Wirkstoffschicht und das Aufbringen der Wirkstoffschicht auf eine feuchte Fläche eines Individuums, woraufhin durch die Aufbringung der Wirkstoff freigesetzt wird, umfasst.

11. Das Verfahren gemäß Anspruch 10, wobei der Wirkstoff ein kosmetischer Wirkstoff ist.

12. Der schmelzextrudierte Film gemäß einem der Ansprüche 1 bis 7 für die Verwendung in einem Verfahren zur Behandlung einer Indikation durch Verabreichung eines Wirkstoffs an ein Individuum, das den genannten Wirkstoff benötigt oder wünscht, wobei der Wirkstoff ein Medikament ist und das Verfahren das Bereitstellen des mehrschichtigen schmelzextrudierten Films, das Entfernen einer oder mehrerer Schutzschicht(en), wahlweise das Zuschneiden des Films zu Einzeldarreichungsformen vor oder nach Entfernen einer oder mehrerer Schutzschicht(en) von der Wirkstoffschicht und das Aufbringen der Wirkstoffschicht auf eine feuchte Fläche eines Individuums, woraufhin durch die Aufbringung der Wirkstoff freigesetzt wird, umfasst.

13. Der schmelzextrudierte Film zur Verwendung gemäß Anspruch 12, wobei die Indikation aus der Gruppe bestehend aus Entzündung, Gicht, Hypercholesterinämie, mikrobielle Infektion, AIDS, Tuberkulose, Pilzinfektion, amöbische Infektion, parasitäre Infektion, Krebs, Tumor, Organabstoßung, Diabetes, Herzinsuffizienz, Arthritis, Asthma, Schmerz, Blutstau, Infektionen im Urinaltrakt, vaginale Infektion, Anfallsleiden, Depression, Psychose, Krampf, Blutgerinnung, Bluthochdruck und Geburtenkontrolle ausgewählt ist.

14. Der schmelzextrudierte Film zur Verwendung gemäß den Ansprüchen 12 oder 13, wobei der Wirkstoff aus der Gruppe bestehend aus 1) Analgetika, bevorzugt aus der Gruppe bestehend aus Aspirin und Ketoprofen, 2) Anästhetika, bevorzugt aus der Gruppe bestehend aus Lidocain und Benzocain, 3) Antiarthritika und Entzündungshemmer, bevorzugt aus der Gruppe bestehend aus Indomethacin, Dexamethason, Ibuprofen, Allopurinol, Hydrocortison, Betamethason, Dexamethason, Prednisolon und Indomethacin, 4) Antiasthmatika, bevorzugt aus der Gruppe bestehend aus Theophyllin, Ephedrin, Beclomethason und Epinephrin, 5) Harntrakt-Desinfektiva, bevorzugt aus der Gruppe bestehend aus Sulfarmethoxazol und Trimethoprim, 6) Antikoagulantien, bevorzugt Heparin, 7) Antikonvulsiva, bevorzugt Diazepam, 8) Antidepressiva, bevorzugt aus der Gruppe bestehend aus Amitriptylin und Imipramin, 9) Mittel, die in der Behandlung von Diabetes und zur Regulation von Blutzucker nützlich sind, 10) Antineoplastika, bevorzugt aus der Gruppe bestehend aus Adriamycin, Fluoruracil und Methotrexat, 11) Antipsychotika, 12) Antihypertensiva, bevorzugt aus der Gruppe bestehend aus Methyldopa, Clonidin, Timolol, Propranolol, Prazosinhydrochlorid und Reserpin, 13) Muskelrelaxanzien, bevorzugt aus der Gruppe bestehend aus Mephalan und Diazepam, 14) Antiprotozoika, bevorzugt aus der Gruppe bestehend aus Chloramphenicol und Trimethoprim, 15) Spermizide, 16) antibakterielle Substanzen, Tetrazykline, Chloramphenicol und Neomycin, 17) Antihistamine und Decongestanzien, bevorzugt aus der Gruppe bestehend aus Chlorpheniramin, Pseudophedrin und Phenylephrin, 18) Verbindungen gegen Parasiten und 19) antivirale Verbindungen, bevorzugt Acyclovir, ausgewählt ist.

## Revendications

1. Une pellicule extrudée à l'état fondu multicouche comprenant
i) une ou plusieurs couches de principe actif produites à partir d'une composition de polymère extrudée à l'état fondu comprenant a) un polymère soluble dans l'eau qui est un oxyde de polyéthylène et b) un principe actif, dans laquelle la quantité du principe actif va de 5 à 60 pour cent, rapporté au poids total de la couche de principe actif i), et la couche de principe actif présente une épaisseur de 0,15 mm ou moins ; et
ii) une ou plusieurs couches protectrices pouvant être retirées pour laisser la ou les couches de principe actif substantiellement intactes, lesdites une ou plusieurs couches protectrices comprenant une polyoléfine en tant que polymère de formation de pellicule.

2. La pellicule extrudée à l'état fondu multicouche de la revendication 1 dans laquelle lesdites une ou plusieurs couches protectrices sont adjacentes à la ou aux couches de principe actif.

3. La pellicule extrudée à l'état fondu multicouche de la revendication 1 dans laquelle une couche d'excipient est agencée entre la ou les couches de principe actif et la ou les couches protectrices.

4. La pellicule extrudée à l'état fondu de n'importe laquelle des revendications 1 à 3 dans laquelle la couche de principe actif présente une épaisseur de 0,05 mm ou moins.

5. La pellicule extrudée à l'état fondu de n'importe laquelle des revendications 1 à 4 dans laquelle la quantité du principe actif va de 20 à 40 pour cent, rapporté au poids total de la couche de principe actif i).

6. La pellicule extrudée à l'état fondu de n'importe laquelle des revendications 1 à 5 dans laquelle lesdites une ou plusieurs couches protectrices comprennent un polymère à base de propylène ou un polymère à base d'éthylène en tant que polymère de formation de pellicule.

7. La pellicule extrudée à l'état fondu de n'importe laquelle des revendications 1 à 6 sous la forme d'une forme de dose unitaire.

8. Un procédé de production d'une pellicule extrudée à l'état fondu comprenant l'étape consistant à coextruder
i) une ou plusieurs couches de principe actif produites à partir d'une composition de polymère extrudée à l'état fondu comprenant a) un polymère soluble dans l'eau qui est un oxyde de polyéthylène et b) un principe actif, dans laquelle la quantité du principe actif va de 5 à 60 pour cent, rapporté au poids total de la couche de principe actif i), et la couche de principe actif présente une épaisseur de 0,15 mm ou moins ;
ii) une ou plusieurs couches protectrices pouvant être retirées pour laisser la ou les couches de principe actif substantiellement intactes, lesdites une ou plusieurs couches protectrices comprenant une polyoléfine en tant que polymère de formation de pellicule ; et
iii) facultativement une couche d'excipient entre la ou les couches de principe actif et la ou les couches protectrices.

9. Le procédé de la revendication 8 comprenant l'étape supplémentaire consistant à découper la pellicule en formes de dose unitaire avant ou après avoir retiré une ou plusieurs couches protectrices de la ou des couches de principe actif.

10. Une méthode non thérapeutique d'administration d'un principe actif à un individu ayant besoin dudit ou souhaitant ledit principe actif, ladite méthode comprenant le fait de fournir la pellicule extrudée à l'état fondu multicouche de n'importe laquelle des revendications 1 à 7, le fait de retirer une ou plusieurs couches protectrices, facultativement le fait de découper la pellicule en formes de dose unitaire avant ou après avoir retiré une ou plusieurs couches protectrices de la couche de principe actif et le fait d'appliquer la couche de principe actif sur une zone humide d'un individu, application suite à laquelle le principe actif est libéré.

11. La méthode de la revendication 10, dans laquelle le principe actif est un principe actif d'un point de vue cosmétique.

12. La pellicule extrudée à l'état fondu de n'importe laquelle des revendications 1 à 7 destinée à une utilisation dans une méthode de traitement d'une indication en administrant un principe actif à un individu ayant besoin dudit ou désirant ledit principe actif, le principe actif étant un médicament et ladite méthode comprenant le fait de fournir la pellicule extrudée à l'état fondu multicouche, le fait de retirer une ou plusieurs couches protectrices, facultativement le fait de découper la pellicule en formes de dose unitaire avant ou après avoir retiré une ou plusieurs couches protectrices de la couche de principe actif et le fait d'appliquer la couche de principe actif sur une zone humide d'un individu, application suite à laquelle le principe actif est libéré.

13. La pellicule extrudée à l'état fondu destinée à une utilisation de la revendication 12, dans laquelle l'indication est sélectionnée dans le groupe constitué d'une inflammation, de la goutte, de l'hypercholestérolémie, d'une infection microbienne, du SIDA, de la tuberculose, d'une infection fongique, d'une infection amibienne, d'une infection parasitaire, d'un cancer, d'une tumeur, d'un rejet d'organe, du diabète, d'une insuffisance cardiaque, de l'arthrite, de l'asthme, d'une douleur, d'une congestion, d'infections des voies urinaires, d'une infection vaginale, d'un trouble lié à une crise, d'une dépression, d'une psychose, d'une convulsion, de la coagulation du sang, de l'hypertension et du contrôle des naissances.

14. La pellicule extrudée à l'état fondu destinée à une utilisation de la revendication 12 ou de la revendication 13, dans laquelle le principe actif est sélectionné dans le groupe constitué 1) d'analgésiques, de préférence dans le groupe constitué de l'aspirine et du ketoprofène, 2) d'anesthésiants, de préférence dans le groupe constitué de la lidocaïne et de la benzocaïne, 3) d'agents anti-arthrite et anti-inflammatoires, de préférence dans le groupe constitué de l'indométacine, du dexaméthasone, de l'ibuprofène, de l'allopurinol, de l'hydrocortisone, de la bétaméthasone, de la dexaméthasone, de la prednisolone et de l'indométacine, 4) de médicaments anti-asthme, de préférence dans le groupe constitué de la théophylline, de l'éphédrine, du béclométhasone et de l'épinéphrine, 5) de désinfectants des voies urinaires, de préférence dans le groupe constitué du sulfaméthoxazole et du triméthoprime, 6) d'anticoagulants, de préférence l'héparine, 7) d'anticonvulsifs, de préférence du diazépam, 8) d'antidépresseurs, de préférence dans le groupe constitué de l'amitriptyline et de l'imipramine, 9) d'agents utiles dans le traitement des diabétiques et dans la régulation de la glycémie, 10) d'antinéoplastiques, de préférence dans le groupe constitué de l'adriamycine, du fluorouracil et du méthotrexate, 11) d'antipsychotiques, 12) d'antihypertenseurs, de préférence dans le groupe constitué du méthyldopa, de la clonidine, du timolol, du propranolol, de l'hydrochlorure de prazosine et de la réserpine, 13) de relaxants musculaires, de préférence dans le groupe constitué du mephalan et du diazépam, 14) d'antiprotozoaires, de préférence dans le groupe constitué du chloramphénicol et du triméthoprime, 15) de spermicides, 16) de substances antibactériennes, de tétracyclines, du chloramphénicol et de la néomycine, 17) d'antihistaminiques et de décongestionnants, de préférence dans le groupe constitué de la chlorphéniramine, de la pseudoéphédrine et de la phényléphrine, 18) de composés antiparasitaires, et 19) de composés antiviraux, de préférence l'acyclovir.
